# EUROPÄISCHE PATENTSCHRIFT

(11) **EP 0 608 516 B1**
(45) Veröffentlichungstag und Bekanntmachung des Hinweises auf die Patenterteilung: **21.01.1998**
(21) Anmeldenummer: 93119481.5
(22) Anmeldetag: 03.12.1993
(51) Int. Cl.: A61B 3/12, A61B 3/135, A61B 3/14

(54) **Ophthalmologisches Gerät**
Ophthalmoscope
Ophtalmoscope

(30) Priorität: 28.01.1993 CH 249/93
(43) Veröffentlichungstag der Anmeldung: 03.08.1994
(73) Patentinhaber: Robert, Yves, Prof. Dr., 8044 Zürich (CH)
(72) Erfinder: Robert, Yves, Prof. Dr., CH-8044 Zürich (CH); Papritz, Franz, CH-3145 Niederscherli (CH); Hendrickson Phillip, Dr., Ch-4054 Basel (CH)
(74) Vertreter: Groner, Manfred, Dr.

(56) Entgegenhaltungen:
- CH-A- 662 261
- DE-A- 3 116 380
- DE-A- 3 818 278
- US-A- 4 251 139

## Beschreibung

Die vorliegende Erfindung bezieht sich auf ein Ophthalmologisches Gerät gemäss dem Oberbegriff des Anpruchs 1.

Solche Geräte haben allgemein den Nachteil, dass es bei wiederholten, späteren Untersuchungen und Messungen für den Arzt fast unmöglich ist, das Gerät mit Bezug auf den Krümmungsmittelpunkt der Corneavorderfläche bzw. die Sehachse des Patientenauges nach Ort und Lage stets genau gleich auszurichten. Dies ist aber notwendig, wenn Untersuchungswerte von zeitlich gestaffelten Untersuchungen vergleichbar sein sollen. Als Beispiel für ein solches Gerät wird auf die CH-PS 662 261 verwiesen. Für eine Augenuntersuchung mit einem solchen Gerät wird der Kopf eines Patienten auf einer Fixationseinrichtung bekannter Art fixiert. Das zu untersuchende Auge seinerseits fixiert der Patient indem er es auf eine in den Strahlengang des Mikroskops eingeblendete Fixationsmarke richtet. Das in Richtung der drei Raumachsen verschiebliche Gerät wird vom Beobachter relativ zum Patientenauge so eingestellt, dass er ein scharfes Bild des Augenhintergrundes bzw. von dessen zu untersuchender Stelle wahrnimmt. Dem Beobachter fehlt indessen die Möglichkeit, bei späteren Untersuchungen das Gerät auf die gleiche Beobachtungsachse wie bei der vorangehenden einzurichten. Dadurch ergeben sich bei nachfolgenden Untersuchungen u.a. andere Lichtreflexionen als bei der vorangegangenen, was die Helligkeitsmessung an der untersuchten Augenhintergrundstelle verfälscht und die Messwerte mangels Reproduzierbarkeit unbrauchbar macht.

Die gattungsbildende US-A-4251139 betrifft ein ophthalmologisches Gerät für optische und fotographische Untersuchungen an einem Patientenauge und weist eine Beleuchtungseinrichtung, eine Beobachtungseinrichtung sowie Mittel auf, um im Bereich der optischen Achse der Beobachtungseinrichtung auf einer zweiten Zwischenbildebene eine Marke zu erzeugen. Diese Marke ist durch eine Verschiebung des Gerätes an eine definierte Stelle verschiebbar. Die Marke wird auf eine Zwischenbildebene B geworfen. Anschliessend wird die Marke zurück in den Beleuchtungsteil des Gerätes gelenkt und von dort abgebildet und kompliziert in den Beobachtungsteil gebracht. Die Marke dient dazu, den achsialen Abstand des Gerätes von der Hornhaut einzustellen.

Die genaue Ausrichtung des Instrumentes auf das Auge erfolgt konkret durch das koachsiale Ausrichten der optischen Achse des Auges einerseits und jener des Beobachtungsinstrumentes anderseits. Eine andere Lösung für ein genaues Ausrichten des Instrumentes ist hier nicht offenbart.

Die Mittel zum koachsialen Ausrichten der optischen Achsen und zur richtigen Distanzierung von Instrument und Patientenauge sind beim Gerät nach der US-A-4251139 vergleichsweise aufwendig. Bei den Beispielen nach den Fig. 1 und 7 sind sie in das Beobachtungsinstrument eingesetzt, speziell Bauteile 20, 21 und F, welche die erforderlichen Lichtpunkte erzeugen und sie in die optische Achse des Beobachtungsinstrumentes einspiegeln. Der damit verbundene konstruktive Aufwand und der dadurch erforderliche Platzbedarf sind unnötig und vergleichsweise gross.

Der Erfindung liegt die Aufgabe zugrunde, die gattungsgemässe Vorrichtung zu vereinfachen ohne dabei die Möglichkeit einer genauen Ausrichtung des Beobachtungsinstrumentes auf das Patientenauge zu beeinträchtigen.

Erfindungsgemäss wird diese Aufgabe gelöst durch die kennzeichnenden Merkmale des Anspruchs 1.

Die Erfindung hat den Vorteil, dass sie auch als Zentrierhilfsmittel bei chirurgischen Eingriffen an einem Patientenauge verwendbar ist, so zum Beispiel zur exakten Zentrierung des Spender- und des Empfängerscheibchens bei der Hornhauttransplantation am Operationsmikroskop.

Bevorzugt weist die erfindungsgemässe Vorrichtung die Merkmale des Anspruchs 2 auf. Dadurch ist es möglich, das Gerät hinsichtlich Ort und Lage zusätzlich exakt reproduzierbar mit Bezug auf die Sehachse des Patientenauges einstellen zu können.

Anhand der beiliegenden schematischen Zeichnung wird die Erfindung beispielsweise erläutert. Es zeigen:
Fig. 1 das Optikschema eines Gerätes und
Fig. 2 das Sehfeld im Okular.

### Beobachtungseinrichtung

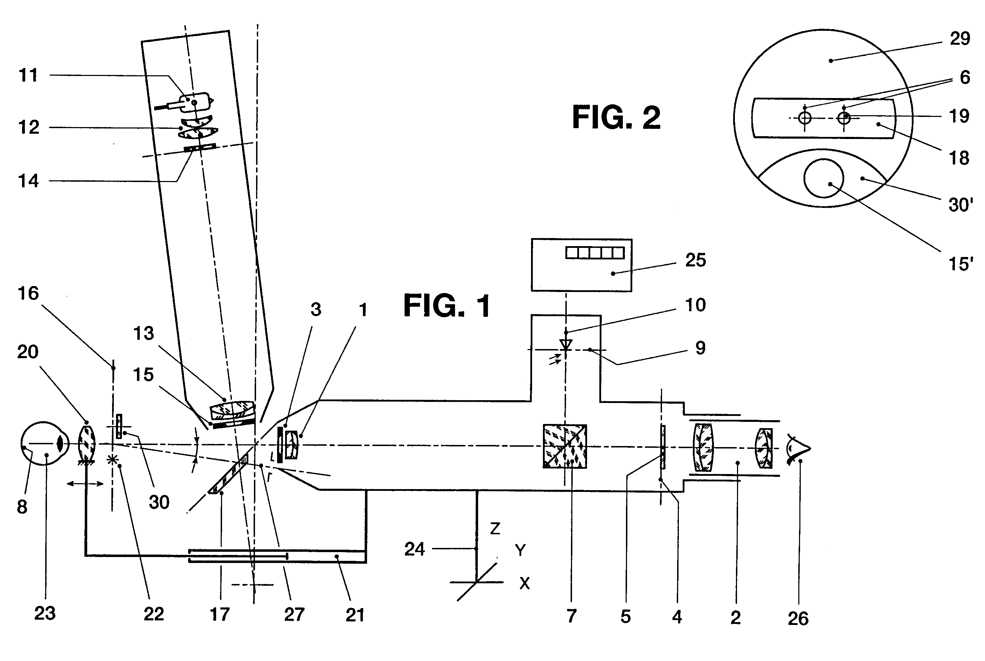

Das Gerät weist ein horizontal angeordnetes 10- bis 20-fach vergrösserndes monokulares Mikroskop mit einem achromatischen Objektiv 1 und einem Okular 2 auf. Das Objektiv 1 ist durch eine Blende 3 in der Öffnung begrenzt. In einer Ebene 4 des Zwischenbildes vor dem Okular 2 ist eine Strichplatte 5 angeordnet, deren Fassung das Sehfeld des Mikroskops begrenzt und welche durch zwei Fadenkreuze 6 in geeignetem Horizontalabstand zwei Bildpunkte markiert. Zwischen dem Objektiv 1 und der Strichplatte 5 ist ein Strahlenteilerwürfel 7 eingefügt, welcher einen Teil des am Augenhintergrund 8 reflektierten Lichts seitlich zu einer Bildebene 9 wegspiegelt und für Messzwecke nutzbar macht. In der seitlichen rechtwinklig zur Zeichenebene orientierten Bildebene 9 sind zwei von einander distanzierte Fotodioden 10 mit kleiner Messfläche derart zentrierbar montiert, dass deren Orte den durch die Fadenkreuze 6 der Strichplatte 5 markierten Bildpunkten exakt entsprechen. In Fig. 1 liegen die beiden Photodioden 10 hintereinander, weshalb nur eine sichtbar ist.

### Beleuchtungseinrichtung

Diese weist als Lichtquelle eine Glüh- oder Halogenlampe 11 mit kleinem Glühkörper, einen Kondensor 12 und eine achromatische Projektionslinse 13 auf. Eingebaut in die Beleuchtungseinrichtung sind weiter eine noch zu beschreibende Filterblende 14 und eine der Projektionslinse 13 vorgesetzte Aperturblende 15.

Aus noch zu erläuternden Gründen ist die Beleuchtungseinrichtung vorzugsweise so angeordnet, dass ihre optische Achse die optische Achse des Mikroskops in deren Zwischenbildebene 16 unter einem Winkel α schneidet. Zweckmässigerweise erfolgt die Beleuchtung von oben über einen unterhalb der Mikroskopachse fest angeordneten Oberflächenspiegel 17, so dass das Licht in bezug auf die optische Achse des Mikroskops von unten unter dem Winkel α einfällt. Die um den Winkel α geneigte Einfallsrichtung des Lichts und eine zweckmässige Dimensionierung der Blenden 3 und 15 dienen dazu, störende Reflexe, verursacht durch Reflexionen an den Oberflächen der brechenden Medien, vom Mikroskopstrahlengang und insbesondere vom Messstrahlengang fernzuhalten. Die Optik der Beleuchtungseinrichtung ist so ausgelegt, dass die Projektionslinse 13 die spezielle Filterblende 14 in der Zwischenbildebene 16 abbildet. Durch einen auf der Filterblende 14 aufgedampften, lichtundurchlässigen Belag oder durch die Ausbildung der Filterblende 14 kann das ausgeleuchtete Feld 18 (Fig. 2) in der Grösse begrenzt werden. In die Zwischenbildebene 16 projiziert soll es eine rechteckige horizontale Ausdehnung haben. Innerhalb des ausgeleuchteten Feldes ist ein Filterbelag angebracht, welcher nach der Art eines Kantenfilters (Langpassfilter) rotes Licht der Wellenlänge von ca. 600 Nanometern und länger durchlässt (analog Wrattenfilter Nr. 25). Dieser Filterbelag bedeckt indessen nicht das ganze ausgeleuchtete Feld 18, sondern lässt um die mit den Fadenkreuzen 6 der Strichplatte 5 bezeichneten Bildpunkte je eine kleine kreisförmige Zone 19 offen für den Durchtritt von "weissem" Licht. Er bezweckt den Patienten nicht übermässig zu blenden, dem Untersucher aber gleichwohl die Orientierung zu ermöglichen.

### Vorsatzlinse

In der Verlängerung der optischen Achse des Mikroskops über die Zwischenbildebene 16 hinaus ist eine asphärische positive Vorsatzlinse 20 nach der Art der "Ophthalmoskopierlinsen für indirekte Ophthalmoskopie" derart angeordnet, dass ihr hinterer (mikroskopseitiger) Brennpunkt primär in oder nahe der Zwischenbildebene 16 liegt, aber mittels einer Gradführung 21 in Achsrichtung auch um einige Millimeter nach beiden Richtungen verschoben werden kann.

### Fixiermarke

In der Zwischenbildebene 16 ist ferner eine rechtwinklig zur Mikroskopachse zweidimensional verschiebliche Fixationsmarke 22 angebracht, die für ein Patientenauge 23 einen Fixierpunkt bildet.

### Instrumentenbasis

Das beschriebene Gerät wird von einer symbolisch dargestellten auf einem Gerätetisch aufliegenden Instrumentenbasis 24 bekannter Bauart getragen und zusammen mit dieser vom Untersucher vor dem Auge des Patienten dreidimensional in Richtung der X-, Y- und Z-Raumachsen ausgerichtet.

### Elektronische Messanzeige

Die Signale der beiden Messdioden 10 werden in einem separaten Auswertegerät 25 rechnerisch verarbeitet und das Ergebnis digital angezeigt.

### Funktion der bisher beschriebenen Elemente

Vorgängig der Untersuchung wird dem Patienten die Pupille erweitert. Der Untersucher reguliert das Okular 2 des Mikroskops für sein Auge 26 akkommodationsfrei auf die eingebaute Strichplatte 5. Wie üblich bei derartigen Untersuchungsgeräten stützt der sitzende Patient seinen Kopf mit Kinn und Stirn auf einer Kopfhalterung ab. Durch den Untersucher wird das Gerät mittels der Einstellelemente der Instrumentenbasis 24 derart ausgerichtet, dass die Vorsatzlinse 20 frontal in einigen Millimetern Abstand vor das Auge 23 des Patienten zu stehen kommt. Bei einigermassen gut zentrierter Ausrichtung ist der Augenhintergrund 8 nun bereits sichtbar. Je nach dem, ob beim Patienten eine Refraktionsanomalie vorliegt, ist die Vorsatzlinse 20 etwas in Richtung der optischen Achse zu verschieben. Bei Miopie zeigt die Verschieberichtung vom Patientenauge weg, bei Hyperopie zu diesem hin. Die Einstellung ist dann richtig, wenn der Untersucher die Netzhaut scharf sieht. Gleichzeitig ist gewährleistet, dass der Patient die in der Zwischenbildebene 16 den Fixierpunkt der verschieblich angeordneten Fixationsmarke 22 scharf wahrnehmen und ihm folgen kann, wenn er vom Untersucher an eine andere Stelle gebracht wird. Auf diese Weise ist es leicht möglich Gerät und Patientenauge 23 so auszurichten, dass die vom Untersucher bevorzugt ausgewählten Netzhautstellen mit den durch die Strichmarken 10 bezeichneten Messpunkten in Übereinstimmung gebracht werden. Mittels der Fotodioden 10 kann nun das von den ausgewählten Netzhautstellen reflektierte Licht gemessen, mit dem Auswertegerät 25 ausgewertet und der oder die Messwert(e) angezeigt werden. Das Auswertegerät 25 bildet u.a. den Quotienten aus den von den beiden Fotodioden 10 ermittelten Helligkeitswerten indem stets der höhere Wert als Dividend und der niedrigere als Divisor eingeht und bringt ihn an einem Display zur Darstellung. Dieser Quotient ist ein Mass für die Kontrastübertragungsfähigkeit der optischen Medien des Patientenauges.

Eine sehr wichtige Voraussetzung um exakt reproduzierbare Messresultate zu erhalten ist indessen mit den beschriebenen Elementen und der angegebenen Arbeitsmethode noch nicht erfüllt. Auf diesen Umstand wird im nachstehenden noch ausführlich eingegangen.

### Mittel zur exakt reproduzierbaren Einstellung des Gerätes

Der einfacheren Darstellung wegen beziehen sich die nachstehenden Ausführungen auf ein emmetropes Patientenauge 23. Bei Einstellung auf das letztere herrscht zwischen dem Auge 23 und der Vorsatzlinse 20 paralleler Strahlengang. Dies hat aber zur Folge, dass leichte Verschiebungen des Gerätes in allen drei Raumachsen weder eine Verschiebung noch eine Schärfenabnahme des Bildes im Okular bewirken. Der Geräteverschiebung wird einzig durch eine dabei einsetzende Vignettierung eine Grenze gesetzt. Je nach der Tiefeneinstellung erfolgt bei einer Querbewegung des Gerätes relativ zum Auge 23 zuerst eine Abnahme der Helligkeit im rechten oder linken Bildteil. Dazwischen gibt es eine exakt definierte Tiefenlage, wo das ganze Bild bei einer Querbewegung gleichmässig in der Helligkeit abgeschwächt wird. Indessen sind sowohl die vom Beobachtungsstrahlengang wie vom Messstrahlengang beanspruchten Strahlenkegel deutlich enger als der durch die erweiterte Augenpupille frei gegebene Raum. Unterschiedliche Messresultate können aber auch entstehen, wenn bei sich folgenden Messungen unterschiedliche Stellen verschiedener Transparenz (Trübung) in den Augenmedien durchstrahlt werden. Deshalb können reproduzierbare Messresultate nur erhalten werden, wenn es gelingt, die Einstellung des Gerätes auch örtlich im Raum der Augenmedien, d.h. in bezug auf die Sehachse und das Krümmungszentrum der Corneavorderfläche exakt reproduzierbar vorzunehmen.

Als Hilfsmittel wird einerseits die Aperturblende 15 der Beleuchtungseinrichtung bzw. deren Spiegelbild 27 beigezogen und anderseits die Vorderfläche der Cornea des Patientenauges als Kugelspiegel benützt. Denkt man sich vorerst das Patientenauge weg, so wird durch die Vorsatzlinse 20 etwas ausserhalb ihres vorderen Brennpunktes ein hell leuchtendes verkleinertes Bild der Aperturblende 15 erzeugt. Ins Auge gebracht kann dieses Blendenbild als sekundäre Lichtquelle für die Beleuchtung der Netzhaut aufgefasst werden. Scharf abgebildet wird dort indessen das ausgeleuchtete, mit einem Rotfilterbelag versehene Feld 18 der Filterblende 14 mit seiner Feldbegrenzung. Wird nun das Gerät derart vor dem Patientenauge 23 ausgerichtet, dass das obige kleine Bild der Aperturblende 15 näherungsweise auf halbe Distanz zwischen Scheitel und Krümmungsmittelpunkt der Corneavorderfläche zu liegen kommt, wird ein Teil des Lichts von deren als Kugelspiegel wirkenden Oberfläche reflektiert und zwar als paralleles Strahlenbündel zur Vorsatzlinse 20 hin, d.h. dass in deren hinterer Brennebene bzw. in der Zwischenbildebene 16 wiederum ein Bild 15' (Fig. 2) der Aperturblende 15 entsteht.

Bei entsprechender Zentrierung des Gerätes ist dieses Bild 15' im Mikroskop als sehr helles scharf begrenztes Kreisbild sichtbar und bei wiederholten Messungen immer wieder an der gleichen Stelle im Sehfeld einstellbar.

Indessen ist dieses Blendenbild 15' verhältnismässig sehr hell und könnte den Messstrahlengang stören. Um dies zu vermeiden wird das Gerät einerseits so zentriert, dass das Blendenbild 15' gegen den Rand des Sehfeldes 29 zu liegen kommt. Zweckmässigerweise liegt es (wie Fig. 2 zeigt) am unteren Rand, wenn das Mikroskop kein bildaufrichtendes System enthält und am oberen, wenn ein solches vorhanden ist. Anderseits wird die Intensität des Blendenbildes 15' durch ein nahe Zwischenbildebene 16 über der optischen Achse angeordnetes lichtschwächendes Filter 30 stark vermindert. Dieses kreisrunde Filter 30 ragt teilweise in das Beobachtungsfeld des Mikroskops (zirka 1/3 des Durchmessers des letzteren) und wird im Okular 2 leicht unscharf wahrgenommen. In diese durch zwei Kreisbogen begrenzte dunkle Zone 30' wird das Blendenbild 15' bei jeder neuen Messung immer wieder eingestellt (Fig. 2). Diese Einstellmethode ist in bezug auf die örtlich-räumliche Wiederholbarkeit der Einstellung sehr empfindlich. Nach kurzer Einübung ist sie nicht schwierig auszuführen und erlaubt gut reproduzierbare Messungen an den gleichen Netzhautstellen zu erhalten.

Ist im Falle anderer Anwendungen der Erfindung nur eine reproduzierbare Einstellbarkeit des Gerätes mit Bezug auf den Krümmungsmittelpunkt der Corneavorderfläche des Patientenauges 23 erforderlich, kann die Fixationsmarke 22 entfallen.

Das Gerät kann als selbständiges einheitliches Gerät gebaut werden, wie auch als aufsetz- oder anbaubares Zusatzgerät zu einem praktisch in jeder Augenarztpraxis vorhandenen Spaltlampengerät. In Kombination mit einem sehr verbreiteten Spaltlampenmodell ist es möglich die Spaltbeleuchtungseinrichtung mit dem beschriebenen Rotfilter auszurüsten, womit eine integrierte Beleuchtungseinrichtung entfällt. In diesem Fall ist auch der Umlenkspiegel 17 bereits vorhanden.

Anstelle der gezeigten Datenerfassungseinrichtung 10, 25 könnte auch eine CCD-Kamera und dgl. mit einer selbsttätigen Auswerteeinrichtung treten, die selektiv an den zwei wichtigsten Bildpunkten die Messung vornimmt, auswertet und anzeigt. Eine weitere Möglichkeit besteht in einer photographischen Aufzeichnung der jeweils untersuchten Netzhautzone zusammen mit einer Markierung der Messpunkte.

## Patentansprüche

1. Ophthalmologisches Gerät für optische und fotographische Untersuchungen an einem Patientenauge
a) mit einer Beleuchtungseinrichtung (11, 12, 13) und
b) einer Beobachtungseinrichtung (1, 2, 3, 9) für den Augenhintergrund bzw. den zu untersuchenden Augenteil,
c) welche Beobachtungseinrichtung (1, 2, 3, 9) dem Patientenauge zugewandt eine Objektivlinse (1) aufweist, der eine erste Zwischenbildebene (4) für die Abbildung des zu untersuchenden Augenteils zugeordnet ist,
d) wobei Mittel (15, 17, 20, 21, 27) vorhanden sind, um im Bereich der optischen Achse der Beobachtungseinrichtung (1, 2, 3, 9) auf einer zweiten Zwischenbildebene (16) eine Marke (27) zu erzeugen,
e) welche Marke durch eine Verschiebung des Gerätes an eine definierte Stelle verschiebbar ist,
dadurch gekennzeichnet,
f) dass vor der Objektivlinse (1) der Beobachtungseinrichtung (1, 2, 3, 9) eine positive Vorsatzlinse (20) nach Art einer Ophthalmoskopierlinse für indirekte Ophthalmoskopie angeordnet ist,
g) dass die zweite Zwischenbildebene (16) im Bereich der dem Patientenauge abgewandten Brennebene der Vorsatzlinse (20) liegt,
h) wobei die Vorsatzlinse (20) in der zweiten Zwischenbildebene (16) ein reelles Zwischenbild des zu untersuchenden Augenteils erzeugt, und
i) dass, wenn das Gerät für eine scharfe Abbildung des zu untersuchenden Augenteils eingerichtet ist, auf einem teildurchlässigen Filter (30) in der zweiten Zwischenbildebene (16) nebst diesem Augenteil gleichzeitig ein scharfes Abbild der Marke (27) entsteht.

2. Gerät nach Anspruch 1, dadurch gekennzeichnet, dass in der Zwischenbildebene (16) ein zweidimensional verstellbarer und für das Patientenauge sichtbarer Fixierpunkt (22) vorhanden ist.

3. Gerät nach Anspruch 1 oder 2, dadurch gekennzeichnet, dass die Beobachtungseinrichtung (1, 2, 3, 9) ein in Richtung der Raumachsen (X, Y, Z) verstellbares Mikroskop ist, welches den zu untersuchenden Teil des Patientenauges vorzugsweise den Augenhintergrund auf zwei Bildebenen (4, 9) abbildet, wobei die erste Bildebene (4) durch ein Okular (2) beobachtbar ist, und dass eine Messvorrichtung (10, 25) mittels einer Fotodetektoranordnung (10) in der zweiten Bildebene (9) Signale erzeugt, die der Intensität des von einer bestimmten Stelle des Patientenauges, vorzugsweise des Augenhintergrundes reflektierten Lichts entsprechen.

4. Gerät nach einem der Ansprüche 1 bis 3, dadurch gekennzeichnet, dass zur Erzeugung der leuchtenden Marke (27) auf der Zwischenbildebene (16) auf der Achse der Beleuchtungseinrichtung (11, 12, 13) eine Aperturblende (15) angeordnet ist.

5. Gerät nach einem der Ansprüche 1 bis 4, dadurch gekennzeichnet, dass die Vorsatzlinse (20) und die Beobachtungseinrichtung (1, 2, 3, 9) gemeinsam auf einer Instrumentenbasis (24) in Richtung mindestens einer der Raumachsen (X, Y, Z) verschiebbar gelagert sind.

6. Gerät nach einem der Ansprüche 1 bis 5, dadurch gekennzeichnet, dass die Vorsatzlinse (20) in Richtung der optischen Achse der Beobachtungseinrichtung (1, 2, 3, 9) verstellbar ist und dass vorzugsweise die Grösse des Verschiebeweges an einer Strichskala ablesbar ist.

7. Gerät nach einem der Ansprüche 1 bis 6, dadurch gekennzeichnet, dass im Bereich der Zwischenbildebene (16) ein teildurchlässiger Filter (30) in das Sichtfeld (29) der Beobachtungseinrichtung (1, 2, 3, 9) hineinragt.

8. Gerät nach einem der Ansprüche 3 bis 7, dadurch gekennzeichnet, dass die Beobachtungseinrichtung (1, 2, 3, 9) eine Strichplatte (5) in der ersten Bildebene (4) mit zwei je einen Bildpunkt bestimmenden Fadenkreuzen (6) aufweist.

9. Gerät nach einem der Ansprüche 4 bis 8, dadurch gekennzeichnet, dass zwischen der Aperturblende (15) und der Lichtquelle (11) der Beleuchtungseinrichtung (11, 12, 13) eine Filterplatte (14) mit einem teildurchlässigen Bereich (18) angeordnet ist, in welchem zwei Zonen (19) grösserer Lichtdurchlässigkeit vorhanden sind, dass die Filterplatte (14) derart mit der Beleuchtungseinrichtung (11, 12, 13) verbunden ist, dass die Zentren der beiden Zonen (19) für einen Beobachter (26) je mit einem der Fadenkreuze (6) der Strichplatte (4) zusammenfallen.

10. Gerät nach Anspruch 9, dadurch gekennzeichnet, dass sich der teildurchlässige Bereich (18) der Filterplatte (14) streifenförmig quer über das Sehfeld (29) der Beobachtungseinrichtung (1, 2, 3, 9) erstreckt.

11. Gerät nach einem der Ansprüche 4 bis 10, dadurch gekennzeichnet, dass die Aperturblende (15) und vorzugsweise auch die Filterplatte (14) mit der Beleuchtungseinrichtung (11, 12, 13) eine an die Beobachtungseinrichtung (1, 2, 3, 9) ankoppelbare Baueinheit bildet.

12. Gerät nach einem der Ansprüche 1 bis 11, dadurch gekennzeichnet, dass die Beleuchtungseinrichtung (11, 12, 13) durch die Beleuchtungseinrichtung einer Spaltlampe gebildet wird und dass die Beobachtungseinrichtung (1, 2, 3, 9) an diese Beleuchtungseinrichtung ankoppelbar ist.

13. Gerät nach einem der Ansprüche 3 bis 11, dadurch gekennzeichnet, dass auf der zweiten Bildebene (9) zwei hinsichtlich ihrer Lage der Lage der Fadenkreuze (6) entsprechende Fotodetektoren (10) vorhanden sind, und dass die Auswerteeinrichtung (10, 25) mindestens einen Dividierer aufweist, der den Quotienten aus den von den beiden Fotodetektoren (10) signalisierten Helligkeitswerten bildet und zur Anzeige bringt.

14. Gerät nach Anspruch 13, dadurch gekennzeichnet, dass die Auswerteeinrichtung Mittel zur fotographischen Aufzeichnung der jeweils untersuchten Netzhautzone zusammen mit einer Markierung der Messpunkte aufweist.

15. Gerät nach einem der Ansprüche 3 bis 11, dadurch gekennzeichnet, dass der zweiten Bildebene eine CCD-Kamera zugeordnet ist, und dass die Auswerteeinrichtung selbsttätig selektiv an den zwei wichtigsten Bildpunkten eine Messung vornimmt, auswertet und anzeigt.

## Claims

1. Ophthalmologic apparatus for optical and photographic examinations of the eye of a patient
a) with an illumination device (11, 12, 13) and
b) an observation device (1, 2, 3, 9) for the rear of the eye or the part of the eye to be examined,
c) which illumination device (1, 2, 3, 9) comprises an objective lens (1) oriented towards the eye of the patient which is associated with a first intermediate image plane (4) for imaging the part of the eye to be examined,
d) and with means (15, 17, 20, 21, 27) for producing a mark (27) in the area of the optical axis of the observation device (1, 2, 3, 9) on a second intermediate image plane (16),
e) which mark is displaceable to a defined point by displacement of the apparatus,
**characterised in that**
f) in front of the objective lens (1) of the observation device (1, 2, 3, 9) is arranged a positive front lens (20) of the type of an ophthalmoscope lens for indirect ophthalmoscopy;
g) the second intermediate image plane (16) lies in the area of the focal plane of the front lens (20) facing away from the eye of a patient;
h) and the front lens (20) produces in the second intermediate image plane (16) a realistic intermediate image of the part of the eye under examination; and
i) when the device is set up for a sharp image of the part of the eye under examination, a sharp image of the mark (27) is simultaneously produced on a partially permeable filter (30) in the second intermediate image plane (16).

2. Apparatus according to Claim 1, **characterised by** the presence a focal point (22), which is two-dimensionally adjustable and visible to the eye of a patient.

3. Apparatus according to Claim 1 or 2, **characterised in that** the observation device (1, 2, 3, 9) is a microscope, which is adjustable in the direction of the space axes (X, Y, Z) and which images the part of a patient's eye under examination, preferably the rear of the patient's eye, on two image planes (4, 9), and the first image plane (4) is observed by an ocular (2), and a measuring device (10, 25) generates by means of a photodetector arrangement (10) in the second image plane (9) signals which correspond with the intensity of light reflected by a specified point of the patient's eye, preferably the rear of the eye.

4. Apparatus according to one of Claims 1 to 3, **characterised in that** an aperture diaphragm (15) is arranged on the axis of the illumination device (11, 12, 13) for the purpose of producing the light-emitting mark (27) on the intermediate image plane (16).

5. Apparatus according to one of Claims 1 to 4, **characterised in that** the front lens (20) and the observation device (1, 2, 3, 9) are jointly mounted on an instrument base (24) so as to be displaceable in the direction of at least one of the space axes (X, Y, Z).

6. Apparatus according to one of Claims 1 to 5, **characterised in that** the front lens (20) is adjustable in the direction of the optical axis of the observation device (1, 2, 3, 9), and preferably the size of the displacement path can be read from a marked dial.

7. Apparatus according to one of Claims 1 to 6, **characterised in that** in the area of the intermediate image plane (16) a partially permeable filter (30) protrudes into the field of vision (29) of the observation device (1, 2, 3, 9).

8. Apparatus according to one of Claims 3 to 7, **characterised in that** the observation device (1, 2, 3, 9) comprises a marked plate (5) in the first image plane (4) with two cross-lines (6) which set a respective image point.

9. Apparatus according to one of Claims 4 to 8, **characterised in that** between the aperture diaphragm (15) and the light source (11) of the illumination device (11, 12, 13) is arranged a filter plate (14) with a part-permeable area (18) with two zones (19) of increased light permeability, and the filter plate (14) is connected to the illumination device (11, 12, 13) in such a manner that the centres of both zones (19) coincide for an observer (26) with respective cross-lines (6) of the marked plate (4).

10. Apparatus according to Claim 9, **characterised in that** the partially permeable area (18) of the filter plate (14) extends in the form of stripes transversely across the field of vision (29) of the observation device (1, 2, 3, 9)

11. Apparatus according to one of Claims 4 to 10, **characterised in that** the aperture diaphragm (15), and preferably also the filter plate (14), together with the illumination device (11, 12, 13) establishes an assembly unit which can be coupled to the observation device (1, 2, 3, 9).

12. Apparatus according to one of Claims 1 to 11, **characterised in that** the illumination device (11, 12, 13) is established by the illumination device of a slit lamp, and the observation device (1, 2, 3, 9) can be coupled to this illumination device.

13. Apparatus according to one of Claims 3 to 11, **characterised in that** on the second image plane (9) are two photo detectors (10) which, relative to their position, correspond with the position of the cross-lines (6), and the evaluation device (10, 25) comprises at least one divider which forms the quotient from brightness values signalled by both photo detectors (10) and displays it.

14. Apparatus according to Claim 13, **characterised in that** the evaluation device comprises means for photographic recording of a respectively examined retina zone together with a marking of the measuring points.

15. Apparatus according to one of Claims 3 to 11, **characterised in that** the second image plane is associated with a CCD camera, and the evaluation device automatically and selectively carries out measuring at the two most important image points, which it evaluates and displays.

## Revendications

1. Appareil ophtalmologique pour des examens optiques et photographiques d'un oeil de patient comprenant :
a) une installation d'éclairage (11, 12, 13) et,
b) une installation d'observation (1, 2, 3, 9) pour le fond de l'oeil ou la partie de l'oeil à examiner,
c) cette installation d'observation (1, 2, 3, 9) ayant, tournée vers l'oeil du patient, un objectif (1) qui comprend un premier diaphragme intermédiaire (4) pour former l'image de la partie de l'oeil à examiner,
d) des moyens (15, 17, 20, 21, 27) étant prévus pour créer au niveau de l'axe optique de l'installation d'observation (1, 2, 3, 9), un repère (27) dans un second plan image intermédiaire (16),
e) ce repère pouvant être déplacé à un emplacement déterminé par coulissement de l'appareil,
caractérisé en ce que
f) en avant de l'objectif (1) de l'installation d'observation (1, 2, 3, 9) il est prévu une lentille additionnelle positive (20) à la manière d'une lentille de copie ophtalmologique pour une ophtalmoscopie indirecte,
g) le second plan image intermédiaire (16) se trouve dans la zone du plan focal de la lentille additionnelle(20), du côté opposé à celui de l'oeil du patient,
h) la lentille additionnelle (20) générant dans le second plan image intermédiaire (16), une image intermédiaire réelle de la partie de l'oeil à examiner et,
i) lorsque l'appareil est prévu pour une copie précise de la partie de l'oeil à examiner, sur un filtre partiellement transparent (30), dans le second plan image intermédiaire (16), on forme à côté de cette partie de l'oeil également une image précise du repère (27).

2. Appareil selon la revendication 1,
caractérisé en ce qu'
un point fixe (22), réglable en dimension et lisible pour l'oeil du patient est prévu dans le plan image intermédiaire (16).

3. Appareil selon les revendications 1 ou 2,
caractérisé en ce que
l'installation d'observation (1, 2, 3, 9) est un microscope réglable en direction des axes de l'espace (X, Y, Z), ce microscope formant une image de la partie à examiner de l'oeil du patient notamment le fond de l'oeil dans deux plans image (4, 9),
le premier plan image (4) pouvant être observé à travers un oculaire (2) et un dispositif de mesure (10, 25) génère par un dispositif à photodétecteurs (10), des signaux dans le second plan image (9), qui correspondent à l'intensité de la lumière réfléchie par un point déterminé de l'oeil du patient de préférence du fond de l'oeil.

4. Appareil selon l'une des revendications 1 à 3,
caractérisé par
un diaphragme d'ouverture (15) pour générer le repère lumineux (27) prévu dans le plan image intermédiaire (16) sur l'axe de l'installation d'éclairage (11, 12, 13).

5. Appareil selon l'une des revendications 1 à 4,
caractérisé en ce que
la lentille additionnelle (20) et l'installation d'observation (1, 2, 3, 9) sont montées coulissantes en commun sur une base d'instrument (24), dans la direction d'au moins l'un des axes de l'espace (X, Y, Z).

6. Appareil selon l'une des revendications 1 à 5,
caractérisé en ce que
la lentille additionnelle (20) est réglable dans la direction de l'axe optique de l'installation d'observation (1, 2, 3, 9) et en ce que de préférence la dimension de la couse se lit sur une échelle graduée.

7. Appareil selon l'une des revendications 1 à 6,
caractérisé en ce qu'
au niveau du plan image intermédiaire (16), un filtre semi-transparent (30) pénètre dans le champ de visée (29) de l'installation d'observation (1, 2, 3, 9).

8. Appareil selon l'une des revendications 3 à 7,
caractérisé en ce que
l'installation d'observation (1, 2, 3, 9) comprend une plaque graduée (5) dans le premier plan image (4) avec deux réticules (6) définissant chacun un point image.

9. Appareil selon l'une des revendications 4 à 8,
caractérisé en ce qu'
entre le diaphragme à ouverture (15) et la source lumineuse (11) de l'installation d'éclairage (11, 12, 13) , il est prévu une plaque de filtre (14) avec une zone (18) semi-transparente ayant deux zones (19) de plus grande transparence à la lumière, et la plaque de filtre (14) est reliée à l'installation d'éclairage (11, 12, 13) pour que le centre des deux zones (19) coïncide pour un observateur (26), chaque fois avec l'un des réticules (6) de la plaque graduée (4).

10. Appareil selon la revendication 9,
caractérisé en ce que
la zone semi-transparente (18) de la plaque de filtre (14) s'étend en forme de bande transversalement par rapport au champ de visée (29) de l'installation d'observation (1, 2, 3, 9).

11. Appareil selon l'une des revendications 4 à 10,
caractérisé en ce que
le diaphragme à ouverture (15) et de préférence également la plaque filtre (14) forment avec l'installation d'éclairage (11, 12, 13), un ensemble susceptible d'être couplé à l'installation d'observation (1, 2, 3, 9).

12. Appareil selon l'une des revendications 1 à 11,
caractérisé en ce que
l'installation d'éclairage (11, 12, 13) est formée par l'installation d'éclairage d'une lampe à fente et l'installation d'observation (1, 2, 3, 9) est couplée à cette installation d'éclairage.

13. Appareil selon l'une des revendications 3 à 11,
caractérisé en ce que
deux photodétecteurs (10) dont la position correspond à celle du réticule (6) sont prévus dans le second plan image (9), et l'installation d'exploitation (10, 25) comporte un diviseur formant le quotient des valeurs de luminosité fournies par les deux photodétecteurs (10) et affiche ses quotients.

14. Appareil selon la revendication 13,
caractérisé en ce que
l'installation d'exploitation comprend des moyens pour l'enregistrement photographique de chaque fois la zone de la peau réticulée, examinée, ainsi qu'un marquage des points de mesure.

15. Appareil selon l'une des revendications 3 à 11,
caractérisé en ce que
le second plan image est associé à une caméra CCD et une installation d'exploitation effectue, exploite et affiche sélectivement d'elle-même une mesure aux deux points image les plus importants.
